(19) Europäisches Patentamt
European Patent Office
Office européen des brevets

(11) EP 1 322 601 B1

(12) **FASCICULE DE BREVET EUROPEEN**

(45) Date de publication et mention de la délivrance du brevet:
**03.08.2005 Bulletin 2005/31**

(21) Numéro de dépôt: **01974423.4**

(22) Date de dépôt: **02.10.2001**

(51) Int Cl.[7]: **C07C 303/28**, C07C 309/66, C07C 41/16, C07C 43/225, B01J 31/02

(86) Numéro de dépôt international:
**PCT/FR2001/003040**

(87) Numéro de publication internationale:
**WO 2002/028826 (11.04.2002 Gazette 2002/15)**

(54) **PROCEDE DE SULFONYLATION D'UN COMPOSE ORGANIQUE HYDROXYLE**

VERFAHREN ZUR SULFONYLIERUNG EINER ORGANISCHEN HYDROXYLVERBINDUNG

METHOD FOR SULPHONYLATING A HYDROXYLATED ORGANIC COMPOUND

(84) Etats contractants désignés:
**AT BE CH CY DE DK ES FI FR GB GR IE IT LI LU MC NL PT SE TR**

(30) Priorité: **03.10.2000 FR 0012583**

(43) Date de publication de la demande:
**02.07.2003 Bulletin 2003/27**

(73) Titulaire: **Rhodia Chimie**
**92512 Boulogne-Billancourt Cedex (FR)**

(72) Inventeurs:
- **VASTRA, Johann**
**F-69700 MONTAGNY (FR)**
- **SAINT-JALMES, Laurent**
**F-69330 Meyzieu (FR)**

(74) Mandataire: **Esson, Jean-Pierre et al**
**Rhodia Services,**
**Direction de la Propriété Industrielle,**
**Centre de Recherches de Lyon**
**BP 62**
**85, avenue des Frères Perret**
**69192 Saint-Fons Cédex (FR)**

(56) Documents cités:
**EP-A- 0 200 657**

- **D. PRESCHER, ET AL.: "Various synthetic approaches to fluoroalkyl p-nitrophenyl ethers" JOURNAL OF FLUORINE CHEMISTRY, vol. 79, no. 2, 1 août 1996 (1996-08-01), pages 145-148, XP004069916 Elsevier Science Publishers, Amsterdam, NL ISSN: 0022-1139**
- **G.A. OLAH, ET AL.: "Preparative carbocation chemistry; VIII. Isolation and sulphonylating ability of sulphonyl halide-antimony pentahalide complexes" SYNTHESIS, no. 5, mai 1974 (1974-05), pages 342-343, XP002169199 Georg Thieme Verlag, Stuttgart, DE ISSN: 0039-7881**
- **G.A. OLAH, ET AL.: "Stabe carbonium ions. CXVI. Sulphonyl halide-antimony pentafluoride complexes and study of protonation and cleavage of sulphonyl halides in fluorosulphuric acid-antimony pentafluoride-sulphuryl chloride fluoride solution" JOURNAL OF ORGANIC CHEMISTRY, vol. 35, no. 11, novembre 1970 (1970-11), pages 3925-3928, XP002169198 American Chemical Society, Washington, DC, US ISSN: 0022-3263**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet un procédé de sulfonylation d'un composé organique hydroxylé. L'invention vise notamment les composés hydroxylés de type aliphatique et plus particulièrement ceux qui comprennent sur leur chaîne aliphatique, un groupe électro-attracteur.

L'invention s'applique préférentiellement aux alcools aliphatiques perfluorés et, notamment, au 2,2,2-trifluoroéthanol.

Il est connu selon Crossland et al de préparer le 2,2,2-trifluoroéthylméthanesulfonate appelé de manière simplifiée « TFEMes » par réaction du 2,2,2-trifluoroéthanol avec le chlorure de mésyle, en présence d'une base telle que la triéthylamine et dans un solvant organique qui est le dichlorométhane.

L'inconvénient dont souffre ce procédé est qu'il est difficilement extrapolable à l'échelle industrielle car il conduit à une faible productivité en raison d'un milieu réactionnel extrêmement dilué et par ailleurs, il conduit à des rejets très polluants dus à la présence de fortes quantités de sel d'ammonium.

Par ailleurs, G. A. OLAH et al. SYNTHESIS, 1974(5), 542-543 ont décrit la préparation d'une arylsulfone selon une réaction de Friedel-Crafts par réaction d'un composé aromatique avec un chlorure d'alkylsufonyle- pentahalogénure d'amoine.

L'objectif de la présente invention est de fournir un procédé permettant d'éviter les inconvénients précités.

Il a maintenant été trouvé et c'est ce qui fait l'objet de la présente invention, un procédé de sulfonylation d'un composé organique hydroxylé présentant au moins un groupe électro-attracteur suffisamment prêt du groupe hydroxyle afin de le désactiver ; le groupe électro-attracteur pouvant être présent sur la chaîne hydrocarbonée linéaire ou cyclique, saturé ou insaturée portant le groupe hydroxyle ou bien présent sur un carbocycle ou hétérocycle aromatique porté par un atome de carbone portant ou proche de celui qui porte le groupe hydroxyle caractérisé par le fait qu'il consiste à faire réagir ledit composé, avec un agent sulfonylant, en présence d'une quantité efficace d'un acide de Lewis qui est un composé comprenant un cation métallique ou métalloïdique accepteur de doublets électroniques considéré comme « intermédiaire » dans la classification « dureté (hard) et mollesse (soft) » définie par R. PEARSON.

Dans le présent texte, « acide de Lewis » signifie un composé comprenant un cation métallique ou métalloïdique accepteur de doublets électroniques, qui réagit avec l'agent sulfonylant plutôt qu'avec le composé organique hydroxylé.

Pour le choix du cation métallique ou métalloïdique, on se réfère à la classification « dureté (hard) et mollesse (soft) » définie par R. PEARSON dans Journal of Chem. Ed. 45, pages 581 - 587 (1968).

On fait appel à des cations métalliques ou métalloïdiques, intermédiaires ou proches de ceux-ci : le terme « intermédiaire » ayant la signification du terme "borderline" utilisé dans cette référence.

Par « intermédiaire », on entend selon l'invention, non seulement tous les cations métalliques ou métalloïdiques classés « intermédiaires » mais aussi tous ceux classés comme durs ou mous à l'exception des cations très durs et des cations très mous.

Par « cation dur », on définit un atome accepteur d'électrons, de petite ou de grande taille et de forte charge positive qui ne contient pas d'électrons non appariés sur l'orbitale de valence. Il s'agit généralement de petits cations de fort degré d'oxydation qui ne possèdent pas d'électrons facilement arrachables.

Comme exemples de cations très durs, on peut citer $B^{3+}$, $Mg^{2+}$, $Al^{3+}$, $Si^{4+}$, $Ti^{4+}$, $Mn^{2+}$, $Fe^{3+}$, $Zr^{4+}$, $La^{3+}$.

Par « cation mou », on définit un atome accepteur d'électrons, de grosse ou de petite taille et de faible charge positive qui contient des électrons non appariés (p ou d) sur l'orbitale de valence. Il s'agit généralement de gros cations de faible degré d'oxydation qui possèdent des électrons facilement arrachables.

Comme exemples de cations très mous, on peut mentionner $Cu^+$, $Ag^+$, $Hg^+$.

Pour le choix d'un cation « intermédiaire » défini selon l'invention, on peut se référer à la littérature et notamment, à l'article de TSE-LOK HO [Chemical Reviews 75 ; n°1, p. 1 - 20, (1975)].

Le cation « intermédiaire » mis en oeuvre dans le procédé de l'invention, présente un degré d'oxydation au moins égal à + 2, de préférence, égal à + 3, + 4 ou + 5.

Comme cations métalliques ou métalloïdique convenant à l'invention, on peut citer particulièrement ceux des éléments métalliques ou métalloïdiques des groupes (IIb), (IVb), (Vb) et (VIb) de la classification périodique des éléments.

Dans le présent texte, on se réfère ci-après à la Classification périodique des éléments publiée dans le Bulletin de la Société Chimique de France, n°1 (1966).

A titre d'exemples de cations convenant bien au procédé de l'invention, on peut citer plus particulièrement parmi ceux du groupe du groupe (IIb), le zinc ; du groupe (IVb), l'étain ; du groupe (Vb), l'antimoine, le bismuth ; du groupe (VIb), le tellure.

Parmi les cations précités, on choisit de préférence : $Zn^{2+}$, $Sn^{2+}$, $Sn^{4+}$, $Sb^{5+}$, $Bi^{3+}$, $Te^{4+}$ et encore plus préférentiellement $Sb^{5+}$.

Pour ce qui est des anions liés à ces cations, leur nature permet d'ajuster la dureté ou la mollesse du cation.

On peut mentionner des anions durs tels que notamment $SO_4^{2-}$, $CH_3COO^-$, $C_6H_5COO^-$, $CH_3SO_3^-$, $CF_3SO_3^-$, $CF_3C_6H_4SO_3^-$ ou des anions intermédiaires tels que $Cl^-$, $Br^-$, $NO_2^-$, $SO_3^{2-}$.

Parmi les anions précités ; on choisit de préférence $Cl^-$ ou $Br^-$.

Comme exemples plus spécifiques d'acides de Lewis, on peut mentionner les sels organiques tels que l'acétate, le propionate, le benzoate, le méthanesulfonate, le trifluorométhanesulfonate des éléments métalliques ou métalloïdiques des groupes précités de la classification périodique des éléments.

En ce qui concerne les sels inorganiques, on peut citer notamment les chlorure, bromure, iodure, sulfate, oxyde et produits analogues des éléments métalliques ou métalloïdiques des groupes précités.

On choisit préférentiellement les halogénures métalliques et plus particulièrement le chlorure ou le bromure d'antimoine (V), d'étain (II) ou (IV), de zinc (II), de bismuth (III) et de tellure (IV).

Il est possible de générer un halogénure in situ et donc, mettre en oeuvre tout composé des éléments précités dans la mesure où il est associé à une source d'halogène.

Ainsi, on peut faire appel au métal ou sous n'importe quelle forme. On peut les apporter sous forme de métal ou d'oxyde ou sous forme saline, sel simple ou double, minéral ou organique. Il est possible de faire appel à un sel minéral de préférence, nitrate, sulfate, oxysulfate, halogénure, oxyhalogénure, silicate, carbonate, oxalate ou à un sel organique de préférence, acétylacétonate ; alcoolate et encore plus préférentiellement méthylate ou éthylate ; carboxylate et encore plus préférentiellement acétate.

En ce qui concerne la source d'halogène, on peut faire appel à tout composé susceptible d'apporter des ions halogène permettant de générer in situ l'halogénure métallique ou métalloïdique.

Comme exemples de sources d'halogène, on peut faire appel à l'halogène sous forme moléculaire; à tout halogénure d'acide minéral ou organique, et plus particulièrement des acides carboxyliques aliphatiques ; à tout sel métallique ou métalloïdique, minéral ou organique susceptible de générer une forme halogénée.

Comme exemples plus spécifiques, on peut mentionner entre autres, le chlore ou le brome : l'acide chlorhydrique, l'acide bromhydrique ; le chlorure d'acétyle ; le chlorure de silicium $SiCl_4$, les halogénosilanes tels que $Me_3SiCl$, $Me_2SiCl_2$, $MeSiCl_3$, $PhMe_2SiCl$, les chlorures de phosphore $PCl_5$ ou $PCl_3$, le chlorure de soufre $SCl_2$.

Selon la forme physique de l'acide de Lewis mis en oeuvre, la catalyse peut être homogène ou hétérogène.

Liquide ou bien soluble dans un solvant, généralement dans l'eau, il peut être mis en oeuvre sous forme supportée en le déposant sur un support minéral ou organique. A cet effet, le support peut être choisi parmi les oxydes de métaux, tels que les oxydes d'aluminium, de silicium, de titane et/ou de zirconium, les argiles et plus particulièrement, le kaolin, le talc ou la montmorillonite ou encore parmi les charbons éventuellement activés par un traitement bien connu à l'acide nitrique, le noir d'acétylène ou les polymères organiques, par exemple les polymères polyvinyliques PVC (polychlorure de vinyle), PVDC (polychlorure de vinylidène) ou polystyréniques qui peuvent être fonctionnalisés avec des fonctions nitrile ou encore polyacryliques (et en particulier utiliser directement le polyacrylonitrile).

Le support peut être sous une forme quelconque, par exemple, poudre, billes, granulés, extrudés...

Le catalyseur supporté peut être préparé selon des techniques connues de l'Homme du métier.

Pour préparer le catalyseur supporté utile à la mise en oeuvre du procédé de la présente invention, on peut recourir à des techniques classiques, connues en elles-mêmes, de préparation de catalyseurs métalliques supportés. On peut se référer, notamment, pour la préparation des différents catalyseurs à l'ouvrage : [J. F. LEPAGE "Catalyse de contact", conception, préparation et mise en oeuvre des catalyseurs industriels, Edition Technip (1978)].

Le catalyseur peut être préparé, par exemple, en introduisant un support dans une solution que l'on prépare en dissolvant au moins un composé approprié du (ou des) éléments(s) choisi(s) ; le dépôt du ou des éléments actifs sur le support est réalisé en distillant le solvant, le plus souvent l'eau et la masse de contact ainsi obtenue est soumise à une opération de séchage.

Selon un autre mode de préparation classique, le dépôt du ou des composés apportant les éléments actifs sur le support est réalisé en précipitant les composés de manière en soi connue et en soumettant la masse de contact ainsi obtenue à un séchage.

Dans la description, on désignera par "catalyseur", le catalyseur constitué par l'acide de Lewis ou bien supporté.

La teneur en phase active représente de 5 à 100 % du poids du catalyseur. Dans un catalyseur supporté, elle représente de 5 à 50 % de préférence, de 5 à 20 % du poids du catalyseur.

Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu.

En ce qui concerne les réactifs mis en oeuvre, le composé organique hydroxylé mis en oeuvre est un composé peu nucléophile. Il présente cette caractéristique dans la mesure où il y a présence d'au moins un groupe électro-attracteur suffisamment prêt du groupe hydroxyle afin de le désactiver. Ainsi, le groupe électro-attracteur peut être présent sur la chaîne hydrocarbonée linéaire ou cyclique, saturé ou insaturée portant le groupe hydroxyle ou bien présent sur un carbocycle ou hétérocycle aromatique porté par un atome de carbone portant ou proche (de préférence

en position $\alpha$ ou $\beta$) de celui qui porte le groupe hydroxyle.

Plus précisément, il répond à la formule (I) :

$$R_1 - O - H \qquad (I)$$

dans ladite formule (I) :

- $R_1$ représente un groupe hydrocarboné, substitué par au moins un groupe électro-attracteur (G), comportant de 1 à 40 atomes de carbone qui peut être un groupe aliphatique acyclique saturé ou insaturé, linéaire ou ramifié ; un groupe carbocyclique ou hétérocyclique saturé, insaturé ou aromatique, monocyclique ou polycyclique; un enchaînement des groupes précités.

Le groupe électro-attracteur (G) est plus particulièrement l'un des atomes ou groupes suivants :

. un groupe acyle ayant de 2 à 20 atomes de carbone, de préférence, acétyle,
. un groupe de formule :

$-X$
$-CX_3$
$-CF_2-CF_3$
$-[CF_2]_p-CF_3$
$-C_p H_a F_b$
$-COOM$
$-COOR_3$
$-CHO$
$-SO_3-M$
$-SO_3-R_3$
$-SO_2-R_3$
$-SO-CF_3$
$-SO_2-CF_3$
$-S-CF_3$
$-NO_2$
$-CN$
$-N=C(R_3)_2$
$-NH-CO-R_3$
$-N^+(R_3)_3$
$-P(O)(OR_3)_2$
$-Si(R_3)_3$

dans lesdites formules, les groupes $R_3$ identiques ou différents représentent un atome d'hydrogène ou un groupe alkyle linéaire ou ramifié, saturé ou insaturé ayant de 1 à 20 atomes de carbone ; X symbolise un atome d'halogène, de préférence un atome de chlore, de brome ou de fluor : M représente un atome de métal alcalin, de préférence le sodium ou le potassium : p représente un nombre allant de 1 à 10, b un nombre allant de 3 à 21 et a + b = 2 p + 1.

Dans le cas où le groupe (G) est porté par un cycle aromatique, il est possible que ce groupe se substitue à un atome d'hydrogène ou soit fixé sur le cycle par l'intermédiaire d'un groupe tel que $-R_2-G$ ; $R_2$ représentant un lien valentiel ou un groupe hydrocarboné divalent, linéaire ou ramifié, saturé ou insaturé, ayant de 1 à 4 atomes de carbone tel que, par exemple, méthylène, éthylène, propylène, isopropylène, isopropylidène.

Parmi les différents groupes précités, les atomes d'halogène, le groupe trifluorométhyle et le groupe nitrile ou nitro sont préférés.

Plus précisément, $R_1$ représente un groupe hydrocarboné ayant de 1 à 20 atomes de carbone qui peut être un groupe aliphatique acyclique saturé ou insaturé, linéaire ou ramifié ; un groupe carbocyclique ou hétérocyclique saturé, insaturé ou aromatique, monocyclique ou polycyclique ; un groupe aliphatique saturé ou insaturé, linéaire ou ramifié, porteur d'un substituant cyclique.

$R_1$ représente préférentiellement un groupe aliphatique acyclique saturé linéaire ou ramifié ayant de préférence de 1 à 12 atomes de carbone, et encore plus préférentiellement de 1 à 4 atomes de carbone.

L'invention n'exclut pas la présence d'une insaturation sur la chaîne hydrocarbonée telle qu'une ou plusieurs

doubles liaisons qui peuvent être conjuguées ou non, ou une triple liaison.

La chaîne hydrocarbonée peut être éventuellement interrompue par un hétéroatome (par exemple, oxygène ou soufre) ou par un groupe fonctionnel dans la mesure où celui-ci ne réagit pas et l'on peut citer en particulier un groupe tel que notamment -CO-.

La chaîne hydrocarbonée peut être éventuellement porteuse d'un ou plusieurs substituants (par exemple, halogène, ester, aldéhyde) dans la mesure où ils n'interfèrent pas avec la réaction de sulfonylation

Le groupe aliphatique acyclique, saturé ou insaturé, linéaire ou ramifié peut être éventuellement porteur d'un substituant cyclique. Par cycle, on entend un cycle carbocyclique ou hétérocyclique, saturé, insaturé ou aromatique.

Le groupe aliphatique acyclique peut être relié au cycle par un lien valentiel, un hétéroatome ou un groupe fonctionnel tels que oxy, carbonyle, carboxy, sulfonyle etc...

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs d'un substituant quelconque dans la mesure où ils ne gênent pas les réactions intervenant dans le procédé de l'invention. On peut mentionner en particulier, les groupes alkyle, alkoxy ayant de 1 à 4 atomes de carbone.

Parmi les groupes aliphatiques porteurs d'un substituant cyclique, on vise plus particulièrement les groupes aralkyle ayant de 7 à 12 atomes de carbone, notamment benzyle ou phényléthyle.

Dans la formule (I), $R_1$ peut également représenter un groupe carbocyclique, saturé ou non ayant de préférence 5 ou 6 atomes de carbone dans le cycle ; un groupe hétérocyclique, saturé ou non, comportant notamment 5 ou 6 atomes dans le cycle dont 1 ou 2 hétéroatomes tels que les atomes d'azote, de soufre et d'oxygène; un groupe carbocyclique ou hétérocyclique aromatique, monocyclique, de préférence, phényle ou pyridyle ou polycyclique condensé ou non, de préférence, naphtyle.

Dès lors que $R_1$ comprend un cycle, celui-ci peut être également substitué. Le nombre de substituants est généralement au plus de 4 par cycle mais le plus souvent égal à 1 ou 2.

Parmi toutes les significations données précédemment pour $R_1$, $R_1$ est de préférence un groupe alkyle linéaire ou ramifié ayant de 1 à 12 atomes de carbone ou un groupe phényle.

Le procédé de l'invention s'applique tout particulièrement aux alcools aliphatiques fluorés et perfluorés répondant à la formule (Ia) :

$$R_1 — O — H \qquad \text{(Ia)}$$

dans ladite formule (Ia), $R_1$ représente une chaîne alkyle fluorée ou perfluorée comprenant de 1 à 10 atomes de carbone et de 1 à 21 atomes de fluor, de préférence, de 3 à 21 atomes de fluor.

Le procédé de l'invention s'applique particulièrement bien aux composés de formule (I) tels que le 2,2,2-trifluoroéthanol, le 2,2-difluoroéthanol, le 1,1-difluoroéthanol, le pentafluoroéthanol, l'hexafluoroisopropanol, le pentafluorophénol, le p-nitrophénol, le p-trifluorométhylphénol.

Pour ce qui est de l'agent sulfonylant, il s'agit d'un composé comprenant au moins un groupe sulfonyle de type $-SO_2R_4$ dans lequel $R_4$ représente un groupe hydrocarboné ayant de 1 à 20 atomes de carbone,

Il répond plus particulièrement à la formule suivante (II) :

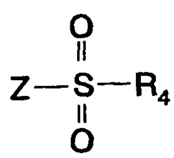
(II)

dans ladite formule (II) :

- $R_4$ représente un groupe hydrocarboné ayant de 1 à 20 atomes de carbone,
- Z représente :
  - . un groupe hydroxyle ou un atome d'halogène, de préférence, un atome de chlore ou de brome,
  - . un groupe $-O-SO_2-R_4'$ dans lequel $R_4'$, identiques ou différents de $R_4$, a la signification donnée pour $R_4$.

Les agents sulfonylants préférés répondent à la formule (II) dans laquelle Z représente un atome de chlore

ou de brome.

Dans la formule (II) ; $R_4$ représente plus particulièrement :

- un groupe alkyle ayant de 1 à 10 atomes de carbone, de préférence, 1 à 4 atomes de carbone et plus préférentiellement un groupe méthyle ou éthyle, éventuellement porteur d'un atome d'halogène, d'un groupe $CF_3$ ou d'un groupe ammonium $N(R_5)_4$, $R_5$, identiques ou différents, représentant un groupe alkyle ayant de 1 à 4 atomes de carbone,
- un groupe cycloalkyle ayant de 3 à 8 atomes de carbone, de préférence, un groupe cyclohexyle,
- un groupe aryle ayant de 6 à 12 atomes de carbone, de préférence, un groupe phényle éventuellement porteur d'un groupe alkyle ayant de 1 à 10 atomes de carbone, de préférence, 1 à 4 atomes de carbone et plus préférentiellement un groupe méthyle ou éthyle, d'un atome d'halogène, d'un groupe $CF_3$ ou d'un groupe $NO_2$,
- un groupe $CX_3$ dans lequel X représente un atome de fluor, de chlore ou de brome,
- un groupe $CF_2$ - $CF_3$,
- un groupe $C_p\ H_a\ F_b$ dans lequel p représente un nombre allant de 1 à 10, b un nombre allant de 3 à 21 et a + b = 2 p + 1.

Les agents sulfonylants préférés répondent à la formule (II) dans laquelle le groupe $-SO_2-\ R_4$ représente :

- les tosyles (p-toluènesulfonyle) $-SO_2-C_6H_4-CH_3$
- les brosyles (p-bromobenzènesulfonyle) $-SO_2-C_6H_4-Br$
- les nosyles (p-nitrobenzènesulfonyle) $-SO_2-C_6H_4-NO_2$
- les mésyles (méthanesulfonyle) $-SO_2-CH_3$
- les bétyles (ammonioalcanesulfonyle) $-SO_2-(CH_2)_nNMe_3^+$ avec n compris entre 0 et 6,
- les triflyles (trifluorométhanesulfonyle) $-SO_2-CF_3$
- les nonaflyles (nonafluorobutanesulfonyle) $-SO_2-C_4F_9$
- les trésyles (2,2,2-trifluroéthanesulfonyle).$-SO_2-CH_2-CF_3$

Comme exemples préférés d'agents sulfonylants, on fait appel plus particulièrement aux composés suivants :

- l'anhydride triflique,
- le chlorure de méthanesulfonyle,
- le chlorure de trifluorométhanesulfonyle,
- le chlorure de benzènesulfonyle,
- le chlorure de p-toluènesulfonyle.

Conformément au procédé de l'invention, on obtient un composé organique sulfonylé par réaction d'un composé organique hydroxylé répondant de préférence, à la formule (I) avec un agent sulfonylant répondant de préférence à la formule (II), en présence d'un acide de Lewis comprenant un cation métallique ou métalloïque M tel que défini ayant un degré d'oxydation n et un contre-ion du cation métallique qui est symbolisé par Y.

L'entité catalytique intervenant dans le procédé de l'invention est réprésentée par les formules (IV) :

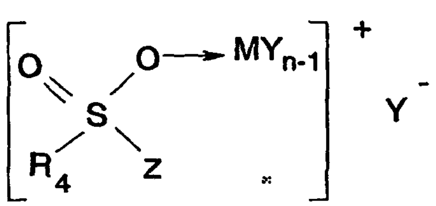

ou

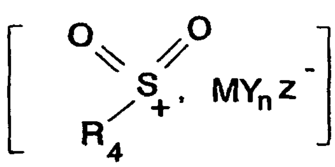

(IV)

dans lesdites formules :

- $R_4$ et Z ont la signification donnée précédemment,
- M symbolise le métal ou le métalloïde au degré d'oxydation n,
- Y représente le contre-ion du cation $M^{n+}$.

La présente invention vise, à titre de produits nouveaux, l'entité catalytique répondant plus précisément aux formules (IVa) :

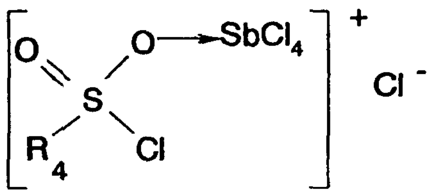

ou

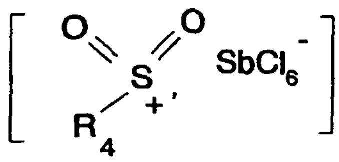

(IVa)

dans lesdites formules :

- $R_4$ a la signification donnée précédemment.

Selon le procédé de l'invention, la réaction entre le composé hydroxylé et l'agent de sulfonylation, est conduite en phase liquide, en présence ou en l'absence d'un solvant organique.

Une variante du procédé de l'invention consiste à conduire la réaction dans un solvant organique.

Plusieurs impératifs président au choix du solvant.

Il doit être inerte dans les conditions de l'invention, et présenter un point d'ébullition supérieur à la température de la réaction.

On fait appel de préférence, à un solvant organique, aprotique et peu polaire.

Comme exemples de solvants convenant à la présente invention, on peut citer en particulier les hydrocarbures aliphatiques ou aromatiques, halogénés ou non.

A titre d'exemples d'hydrocarbures aliphatiques, on peut citer plus particulièrement les paraffines tels que notamment, l'hexane, l'heptane, l'octane, le nonane, le décane, le undécane, le dodécane, le tétradécane ou le cyclohexane et les hydrocarbures aromatiques et plus particulièrement les hydrocarbures aromatiques comme notamment le benzène, le toluène, les xylènes, le cumène, les coupes pétrolières constituées de mélange d'alkylbenzènes notamment les coupes de type Solvesso®.

En ce qui concerne les hydrocarbures halogénés aliphatiques ou aromatiques, on peut mentionner plus particulièrement, les hydrocarbures perchlorés tels que notamment le tétrachlorométhane, le tétrachloroéthylène, l'hexachloroéthane ; les hydrocarbures partiellement chlorés tels que le dichlorométhane, le chloroforme, le 1,2-dichloroéthane, le 1,1,1-trichloroéthane, le 1,1,2,2-tétrachloroéthane, le pentachloroéthane, le trichloroéthylène, le 1-chlorobutane, le 1,2-dichlorobutane ; le monochlorobenzène, le 1,2-dichlorobenzène, le 1,3-dichlorobenzène, le 1,4-dichlorobenzène, le 1,2,4-trichlorobenzène ou des mélanges de différents chlorobenzènes ; le bromoforme, le bromoéthane ou le 1,2-dibromoéthane ; le monobromobenzène ou des mélanges de monobromobenzène avec un ou plusieurs dibromobenzènes ; le 1-bromonaphtalène.

On peut également utiliser un mélange de solvants organiques.

Les solvants préférés sont : le dichlorométhane ou le toluène.

Comme mentionné précédemment, le composé hydroxylé est mis à réagir avec un agent de sulfonylation, éventuellement en présence d'un solvant réactionnel tel que défini et en présence d'un catalyseur de type acide de Lewis.

Le rapport entre le nombre de moles d'agent de sulfonylation et le nombre de moles de composé hydroxylé peut varier entre 0,9 et 2, et se situe de préférence entre 1,0 et 1,2.

La quantité de catalyseur que l'on met en oeuvre dans le procédé de l'invention peut varier dans de larges limites. Elle peut représenter en poids par rapport au composé hydroxylé engagé, de 0,01 à 20 %, de préférence, de 0,05 à 10 %, et encore plus préférentiellement entre 0,1 et 2 %.

Dans le cas où l'on met en oeuvre un solvant organique, sa quantité mise en oeuvre est choisie généralement de telle sorte que la concentration du produit obtenu varie entre 10 et 60 %, de préférence, entre 20 et 30 %.

La température à laquelle est mise en oeuvre la réaction de sulfonylation dépend de la réactivité du substrat de départ et de celle de l'agent de sulfonylation.

Elle se situe entre 20°C et 150°C, de préférence entre 70°C et 100°C.

Généralement, la réaction est conduite à pression atmosphérique mais des pressions plus faibles ou plus élevées peuvent également convenir. On travaille sous pression autogène lorsque la température de réaction est supérieure à la température d'ébullition des réactifs et/ou des produits.

Selon une variante préférée du procédé de l'invention, on conduit le procédé de l'invention, sous atmosphère contrôlée de gaz inertes. On peut établir une atmosphère de gaz rares, de préférence l'argon mais il est plus économique de faire appel à l'azote.

D'un point de vue pratique, le procédé peut être mis en oeuvre en discontinu ou en continu.

Selon une première variante, on charge l'agent de sulfonylation et le catalyseur de type acide de Lewis.

Après mise en contact des réactifs, on porte sous agitation, le mélange réactionnel à la température souhaitée.

On additionne ensuite, le composé hydroxylé, de préférence, progressivement.

On laisse sous agitation jusqu'à consommation complète des réactifs qui peut être suivie par méthode analytique, par exemple chromatographie en phase gazeuse.

En fin de réaction, on récupère une phase liquide comprenant le composé sulfonylé qui peut être récupéré de manière classique, par exemple, par distillation ou par cristallisation.

Après séparation du composé sulfonylé par distillation, on obtient un pied de distillation comprenant le catalyseur qui peut être recyclé plusieurs fois.

On peut envisager également en fin de réaction, de séparer le catalyseur. S'il est insoluble, on peut le séparer selon une technique de séparation solide/liquide de préférence, par filtration.
Dans le cas d'un catalyseur soluble, on l'élimine en traitant le milieu avec un complexant, par exemple, l'acide tartrique ou le carbonate de sodium.

Dans le cas de la préparation du 2,2,2-trifluoroéthylméthanesulfonate, à partir du 2,2,2-trifluoroéthanol et du chlorure de mésyle, on piège l'acide chlorhydrique formé dans une colonne de base, de préférence, la soude puis on récupère le produit formé par distillation.

L'autre variante de l'invention consiste à conduire la réaction en continu, dans un réacteur tubulaire comportant le catalyseur solide disposé en lit fixe.

Le composé hydroxylé et l'agent de sulfonylation peuvent être introduits séparément ou en mélange dans le réacteur.

Ils peuvent également être introduits dans un solvant tel que mentionné précédemment.

On traite la phase liquide obtenu comme mentionné précédemment.

On obtient un composé sulfonylé répondant à la formule (III) :

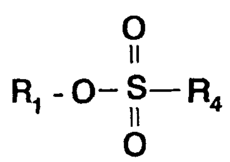

**(III)**

dans ladite formule (III), $R_1$ et $R_4$ ont la signification donnée précédemment.

Le composé de formule (III) peut être utilisé comme produit intermédiaire pour la préparation d'éthers-oxydes.

Ainsi, le composé (III) permet de fixer un groupe $R_1$ sur un composé organique hydroxylé, (en particulier sur un composé aromatique hydroxylé) et qui peut être symbolisé par la formule (V) :

$$R_6 — O — H \quad (V)$$

dans ladite formule $R_6$ a les significations données pour $R_1$, sans toutefois nécessité la présence d'un groupe électro-attracteur.

Un autre objet de l'invention réside en un procédé de préparation d'un composé éther-oxyde de formule (VI) :

$$R_6 — O — R_1 \quad (VI)$$

dans ladite formule $R_6$, identique ou différent de $R_1$, a les significations données pour $R_1$, sans toutefois nécessité la présence d'un groupe électro-attracteur caractérisé par le fait qu'il consiste à préparer un composé de formule (III) obtenu selon l'invention puis à faire réagir le produit obtenu avec un composé hydroxylé de formule (V), en présence d'une base.

L'invention permet d'obtenir des éthers-oxydes symétriques si $R_6$ est identique à $R_1$ ou des éthers-oxydes

asymétriques si $R_6$ est différent de $R_1$.

Selon l'invention, on effectue plus particulièrement la préparation d'un composé de formule (VI) dans laquelle $R_6$ représente un cycle aromatique et qui répond à la formule suivante :

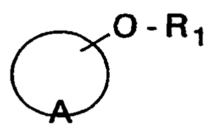

(VIa)

dans ladite formule (VIa), $R_1$ a la signification donnée précédemment et A symbolise le reste d'un cycle formant tout ou partie d'un système carbocyclique ou hétérocyclique, aromatique, monocyclique ou polycyclique

L'invention s'applique notamment aux composés aromatiques répondant à la formule (VIa) dans laquelle A est le reste d'un composé cyclique, ayant de préférence, au moins 4 atomes dans le cycle, éventuellement substitué, et représentant au moins l'un des cycles suivants :

- un carbocycle aromatique, monocyclique ou polycyclique,
- un hétérocycle aromatique, monocyclique ou polycyclique comportant au moins un des hétéroatomes O, N et S,

On précisera, sans pour autant limiter la portée de l'invention, que le reste A éventuellement substitué représente, le reste :

1° - d'un composé carbocyclique aromatique, monocyclique ou polycyclique.
Par "composé carbocyclique polycyclique", on entend :

. un composé constitué par au moins 2 carbocycles aromatiques et formant entre eux des systèmes ortho- ou ortho- et péricondensés,
. un composé constitué par au moins 2 carbocycles dont l'un seul d'entre eux est aromatique et formant entre eux des systèmes ortho- ou ortho- et péricondensés.

2° - d'un composé hétérocyclique aromatique, monocyclique ou polycyclique.
Par "composé hétérocyclique polycyclique", on définit :

. un composé constitué par au moins 2 hétérocycles contenant au moins un hétéroatome dans chaque cycle dont au moins l'un des deux cycles est aromatique et formant entre eux des systèmes ortho- ou ortho- et péricondensés,
. un composé constitué par au moins un cycle hydrocarboné et au moins un hétérocycle dont au moins l'un des cycles est aromatique et formant entre eux des systèmes ortho- ou ortho- et péricondensés.

3° - d'un composé constitué par un enchaînement de cycles, tels que définis aux paragraphes 1 et/ou 2 liés entre eux par un lien valentiel, par un groupe alkylène ou alkylidène ayant de 1 à 4 atomes de carbone, de préférence un groupe méthylène ou isopropylidène ou un atome ou groupes tels que l'oxygène, un groupe carbonyle ou un groupe carboxy etc...

Plus particulièrement, le reste A éventuellement substitué représente, le reste :

- d'un composé monocyclique, carbocyclique, aromatique, tel que par exemple, le benzène,
- d'un composé polycyclique, condensé, aromatique, tel que par exemple, le naphtalène,
- d'un composé polycyclique, non condensé, carbocyclique, aromatique, tel que par exemple, le phénoxybenzène,
- d'un composé polycyclique, condensé, carbocyclique, partiellement aromatique, tel que par exemple, le tétrahydronaphtalène, le 1,2-méthylène dioxybenzène,
- d'un composé polycyclique, non condensé, carbocyclique, partiellement aromatique, tel que par exemple, le cyclohexylbenzène,
- d'un composé monocyclique, hétérocyclique, aromatique, tel que par exemple, la pyridine, le furane, le thiophène,
- d'un composé polycyclique, condensé, aromatique, partiellement hétérocyclique, tel que, par exemple, la quinoléïne, l'indole ou le benzofurane,

- d'un composé polycyclique, non condensé, aromatique, partiellement hétérocyclique, tel que, par exemple, les phénylpyridines, les naphtylpyridines,
- d'un composé polycyclique, condensé, partiellement aromatique, partiellement hétérocyclique, tel que par exemple, la tétrahydroquinoléïne,
- d'un composé polycylique, non condensé, partiellement aromatique, partiellement hétérocyclique, tel que par exemple, la cyclohexylpyridine.

L'invention n'exclut pas la présence d'un substituant sur le cycle d'une nature quelconque dans la mesure où il ne gêne pas la réaction. On peut citer entre autres les groupes alkyle ou alkoxy ayant de 1 à 4 atomes de carbone.

La réaction du composé de formule (V) et du composé de formule (III) est une réaction de O-alkylation qui est effectuée en présence d'une base.

Intervient donc, dans le procédé de l'invention, une base qui peut être minérale ou organique.

On choisit préférentiellement une base forte c'est-à-dire une base ayant un pka supérieur ou égal à 10 : le pKa étant défini comme le cologarithme de la constante de dissociation de l'acide conjuguée mesurée, en milieu aqueux, à 25°C.

Conviennent particulièrement bien à la mise en oeuvre du procédé de l'invention, les bases minérales telles que les sels de métaux alcalins, de préférence, un hydroxyde de métal alcalin qui peut être l'hydroxyde de sodium ou de potassium ; un carbonate de métal alcalin, de préférence, de potassium.

Il est également possible de faire appel à un hydroxyde d'ammonium quaternaire.

Comme exemples d'hydroxyde d'ammonium quaternaire, on met en oeuvre préférentiellement, les hydroxydes de tétralkylammonium ou de trialkylbenzylammonium dont les groupes alkyle identiques ou différents représentent une chaîne alkyle linéaire ou ramifiée ayant de 1 à 12 atomes de carbone, de préférence de 1 à 6 atomes de carbone. On choisit préférentiellement l'hydroxyde de tétraméthylammonium, l'hydroxyde de tétraéthylammonium, l'hydroxyde de tétrabutylammonium ou l'hydroxyde de triméthylbenzylammonium.

On peut également mettre en oeuvre un alcoolate métallique par exemple, le méthylate de sodium ou de potassium, le tert-butylate de potassium.

Pour des considérations économiques, on choisit parmi toutes les bases, préférentiellement l'hydroxyde de sodium ou le carbonate de potassium.

La concentration de la solution basique de départ n'est pas critique. La solution d'hydroxyde de métal alcalin mise en oeuvre a une concentration généralement comprise entre 10 et 50 % en poids.

La quantité de base introduite dans le milieu réactionnel est telle que le rapport entre le nombre de moles de base exprimée en $OH^-$ et le nombre de moles de composé (V) varie entre 1 et 2, de préférence, entre 1 et 1,5.

La réaction a lieu en présence d'un solvant organique, de préférence, un solvant organique polaire.

Comme exemples de solvants organiques aprotiques, polaires qui peuvent également être mis en oeuvre dans le procédé de l'invention, on peut citer plus particulièrement les composés nitrés comme par exemple, le nitrométhane, le nitroéthane, le 1-nitropropane, le 2-nitropropane ou leurs mélanges, le nitrobenzène ; les nitriles alphatiques ou aromatiques comme l'acétonitrile, le propionitrile, le benzonitrile, le cyanure de benzyle ; les carboxamides linéaires ou cycliques comme le N,N-diméthylacétamide (DMAC), le N,N-diéthylacétamide, le diméthylformamide (DMF), le diéthylformamide ou la 1-méthyl-2-pyrrolidinone (NMP) ; le diméthylsulfoxyde (DMSO) ; la tétraméthylènesulfone (sulfolane) ; l'hexaméthylphosphotriamide (HMPT).

On peut utiliser également à titre de solvants organiques, les éther-oxydes aliphatiques, cycloaliphatiques ou aromatiques et, plus particulièrement, l'oxyde de dipropyle, l'oxyde de diisopropyle, l'oxyde de dibutyle, le méthyltertiobutyléther, le diméthyléther de l'éthylèneglycol (ou glyme), le diméthyléther du diéthylèneglycol (ou diglyme) ; l'oxyde de phényle ; le dioxane, le tétrahydrofurane (THF).

Les solvants préférés sont le DMAC et le DMF.

On peut également utiliser un mélange de solvants organiques.

Pour ce qui est des différents réactifs et substrats, ils sont mis en oeuvre dans les quantités précisées ci-après.

La concentration du composé de formule (V) de préférence, (Va) mis en oeuvre dans le solvant peut varier dans de très larges limites. Généralement, la concentration dudit composé varie entre 20 et 30 % du poids du solvant.

La réaction de basification a lieu entre 0°C et 60°C, de préférence, entre 0°C et 20°C.

Elle est conduite généralement sous pression atmosphérique.

D'un point de vue pratique, on prépare le sel du composé de formule (V) en le faisant réagir avec la base.

On élimine l'eau formée puis l'on additionne le composé sulfonylé.

La réaction de O-alkylation a lieu entre 60°C et 160°C, de préférence, entre 120°C et 140°C.

On obtient le composé O-alkylé de formule (VI).

On évapore le solvant réactionnel et l'on récupère le produit obtenu d'une manière classique, par exemple par distillation ou cristallisation.

On donne ci-après des exemples de réalisation de l'invention qui sont donnés à titre illustratif et sans caractère

limitatif.

$$\text{Rendement (RR)} = \frac{\text{nombre de moles de composé sulfonylé formées}}{\text{nombre de moles de composé hydroxylé introduites}} (\%)$$

Exemple 1:

Préparation du 2,2,2-trifluoroéthylméthanesulfonate (TFEMes) selon un procédé semi-continu :

Dans le réacteur sont successivement chargés : 600 g de chlorure de mésyle (CMS, composé de référence) et 24 g de $SbCl_5$.
Le mélange est agité et chauffé à 75 - 85°C.
On ajoute ensuite lentement 552 g de 2,2,2-trifluoroéthanol (TFE) tout en maintenant la température entre 75°C et 80°C.
Le mélange réactionnel est agité 2h supplémentaires environ après l'addition du TFE.
Le milieu réactionnel brut est ensuite ramené à la température ambiante avant d'être distillé sous pression réduite (20-50 mbar, point d'ébullition du TFEMes = 99°C/35 mm Hg).
Après distillation, on obtient 828 g de TFEMes, soit un rendement de 89 %.

Exemple 2:

Préparation du 2,2,2-trifluoroéthylméthanesulfonate (TFEMes) selon un procédé semi-continu :

Dans le réacteur sont successivement chargés : 600 g de 2,2,2-trifluoroéthanol (TFE, composé de référence) et 90 g de $ZnCl_2$.
Le mélange est agité et chauffé à 75 - 85°C.
On ajoute ensuite lentement 738 g de chlorure de mésyle (CMS) tout en maintenant la température entre 75°C et 85°C pendant 13 heures environ.
Le milieu réactionnel brut est ensuite ramené à la température ambiante avant d'être distillé sous pression réduite (20-50 mbar, point d'ébullition TFEMes = 99°C/35 mm Hg).
Après distillation, on obtient 714 g de TFEMes , soit un rendement de 67%.

Exemple 3:

Préparation du 2,2,2-trifluoroéthylméthanesulfonate (TFEMes) selon un procédé discontinu :

Dans le réacteur sont successivement chargés : 750 g de chlorure de mésyle (CMS), 96 g de $TeCl_4$ et 600 g de 2,2,2-trifluoroéthanol (TFE, composé de référence).
Le mélange est agité et chauffé à 75-85°C pendant 9 heures environ.
Le milieu réactionnel brut est ensuite ramené à la température ambiante avant d'être distillé sous pression réduite (20-50 mbar, point d'ébullition TFEMes = 99°C/35 mm Hg).
Après distillation, on obtient 450 g de TFEMes, soit un rendement de 42 %.

Exemple 4

Préparation de l'oxyde de phényle et de 2,2,2-trifluoroéthyle :

A partir du 2,2,2-trifluoroéthylméthanesulfonate (TFEMes) préparé indifféremment selon les exemples 1-3, les éthers comme l'oxyde de phényle et de 2,2,2-trifluoroéthyle peuvent être obtenus de la façon suivante :
Dans un réacteur, 600 g de phénol (composé de référence) sont dissous dans 4920 g de diméthylacétamide (DMAC).
La solution obtenue est chauffée à 50°C puis on additionne lentement 1,3 g d'une solution aqueuse de KOH à 50 % en poids.
Le mélange est maintenu à 60°C pendant 30 min environ après l'addition de la solution de KOH.
L'eau formée est évaporée sous pression réduite.
On additionne ensuite lentement (environ 2 heures) au mélange réactionnel, 1236 g de TFEMes tout en maintenant la température du mélange réactionnel entre 120°C et 140°C.
A la fin de l'addition l'agitation et la température (120°C - 140°C) sont maintenus pendant environ 3 heures.

Le solvant est ensuite distillé sous pression réduite et le concentrat est repris dans 3600 g d'eau et 3000 g de toluène.
La phase organique est ensuite lavée avec 3600 g d'eau, séchée sur $K_2CO_3$, filtrée puis les solvants sont évaporés sous pression réduite.
Le concentrat titre 80-85 % en poids du produit attendu.

Exemple 5

Préparation du 1,1,1,3,3,3-hexafluoroisopropylméthanesulfonate (HFIPMes) selon un procédé discontinu :

Dans le réacteur, sont successivement chargés : 600 g de chlorure de mésyle (CMS, composé de référence) et 24 g de $SbCl_5$ et 880 g de 1,1,1,3,3,3-hexafluoro-2-propanol (HFIP).
Le mélange est agité et chauffé à 55°C pendant 9 heures environ.
Le milieu réactionnel brut est ensuite ramené à la température ambiante avant d'être distillé sous pression réduite (20-50 mbar, point d'ébullition HFIPMes = 27°C/1 mm Hg).
Après distillation, on obtient 1,160 g de HFIPMes, soit un rendement de 90%.

Exemple 6

Préparation du 2,2,2-trichloroethylméthanesulfonate (TCEMes) selon un procédé discontinu :

Dans le réacteur, sont successivement chargés : 600 g de chlorure de mésyle (CMS, composé de référence) et 24 g de $SbCl_5$ et 783 g de 2,2,2-trichloroéthanol (TCE).
Le mélange est agité et chauffé à 85-90°C pendant 8 heures environ.
Le dosage du milieu réactionnel brut par chromatographie en phase gaz avec étalon interne indique que le rendement en TCEMes est de 48 %.

Exemple 7

Préparation du 2,2-difluoroéthylméthanesulfonate (DFEMes) selon un procédé discontinu :

Dans le réacteur sont successivement chargés : 6 g de chlorure de mésyle (CMS, composé de référence) et 0,24 g de $SbCl_5$ et 4,3 g de 2,2-difluoroéthanol (DFE).
Le mélange est agité et chauffé à 80-85°C pendant 8 heures environ.
Le dosage du milieu réactionnel brut par chromatographie en phase gaz avec étalon interne indique que le rendement en DFEMes est de 11 %.

Exemple 8

Préparation du 2-fluoroethylméthanesulfonate (MFEMes) selon un procédé discontinu :

Dans le réacteur, sont successivement chargés : 6 g de chlorure de mésyle (CMS, composé de référence) et 0,24 g de $SbCl_5$ et 6 g de 3,3,3-trifluoropropanol (TFP).
Le mélange est agité et chauffé à 85-90°C pendant 8 heures environ.
Le dosage du milieu réactionnel brut par chromatographie en phase gaz avec étalon interne indique que le rendement en MFEMes est de 44 %.

Exemple 9

Préparation du 3,3,3-trifluoropropylméthanesulfonate (mNPMes) selon un procédé discontinu :

Dans le réacteur, sont successivement chargés : 6 g de chlorure de mésyle (CMS, composé de référence) et 0,24 g de $SbCl_5$ et 7.3 g de 3-nitrophénol (mNP).
Le mélange est agité et chauffé à 85-90°C pendant 8 heures environ.
Le dosage du milieu réactionnel brut par chromatographie en phase gaz avec étalon interne indique que le rendement en mNPMes est de 34 %.

## Revendications

1. Procédé de sulfonylation d'un composé organique hydroxylé présentant au moins un groupe électro-attracteur suffisamment prêt du groupe hydroxyle afin de le désactiver ; le groupe électro-attracteur pouvant être présent sur la chaîne hydrocarbonée linéaire ou cyclique, saturé ou insaturée portant le groupe hydroxyle ou bien présent sur un carbocycle ou hétérocycle aromatique porté par un atome de carbone portant ou proche de celui qui porte le groupe hydroxyle **caractérisé par le fait qu'**il consiste à faire réagir ledit composé, avec un agent sulfonylant, en présence d'une quantité efficace d'un acide de Lewis qui est un composé comprenant un cation métallique ou métalloïdique accepteur de doublets électroniques considéré comme « intermédiaire » dans la classification « dureté (hard) et mollesse (soft) » définie par R. PEARSON.

2. Procédé selon la revendication 1 **caractérisé par le fait que** le cation « intermédiaire » mis en oeuvre présente un degré d'oxydation au moins égal à + 2, de préférence, égal à + 3, + 4 ou + 5.

3. Procédé selon l'une des revendications 1 et 2 **caractérisé par le fait que** le cation est un cation métallique ou métalloïdique des éléments métalliques ou métalloïdiques des groupes (IIb), (IVb), (Vb) et (VIb) de la classification périodique des éléments.

4. Procédé selon l'une des revendications 1 à 3 **caractérisé par le fait que** le cation est choisi parmi les cations suivants : $Zn^{2+}$, $Sn^{2+}$, $Sn^{4+}$, $Sb^{5+}$, $Bi^{3+}$, $Te^{4+}$ et encore plus préférentiellement $Sb^{5+}$.

5. Procédé selon l'une des revendications 1 à 4 **caractérisé par le fait que** l'acide de Lewis est un composé comprenant un anion choisi parmi les anions suivants : anions durs tels que $SO_4^{2-}$, $CH_3COO^-$, $C_6H_5COO^-$, $CH_3SO_3^-$, $CF_3SO_3^-$, $CF_3C_6H_4SO_3^-$ ou des anions intermédiaires tels que $Cl^-$, $Br^-$, $NO_2^-$, $SO_3^{2-}$.

6. Procédé selon la revendication 5 **caractérisé par le fait que** l'anion est choisi parmi $Cl^-$ ou $Br^-$.

7. Procédé selon l'une des revendications 1 à 6 **caractérisé par le fait que** l'acide de Lewis est un sel organique tel que l'acétate, le propionate, le benzoate, le méthanesulfonate, le trifluorométhanesulfonate des éléments métalliques ou métalloïdiques des groupes précités de la classification périodique des éléments et/ou un sel inorganique tel que les chlorure, bromure, iodure, sulfate, oxyde et produits analogues des éléments métalliques ou métalloïdiques des groupes précités.

8. Procédé selon la revendication 1 **caractérisé par le fait que** l'acide de Lewis est choisi parmi les halogénures métalliques et plus préférentiellement le chlorure ou le bromure d'antimoine (V), d'étain (II) ou (IV), de zinc (II), de bismuth (III) et de tellure (IV).

9. Procédé selon l'une des revendications 1 à 8 **caractérisé par le fait que** le catalyseur est déposé sur un support minéral ou organique.de préférence, choisi parmi les oxydes de métaux, tels que les oxydes d'aluminium, de silicium, de titane et/ou de zirconium, les argiles et plus particulièrement, le kaolin, le talc ou la montmorillonite ou encore parmi les charbons éventuellement activés par un traitement à l'acide nitrique, le noir d'acétylène ou les polymères organiques, de préférence, les polymères polyvinyliques ou polystyréniques.

10. Procédé selon l'une des revendications 1 à 9 **caractérisé par le fait que** le composé organique hydroxylé répond à la formule (I) :

$$R_1 — O — H \qquad (I)$$

dans ladite formule (I) :

- $R_1$ représente un groupe hydrocarboné, substitué par au moins un groupe électro-attracteur (G), comportant de 1 à 40 atomes de carbone qui peut être un groupe aliphatique acyclique saturé ou insaturé, linéaire ou ramifié ; un groupe carbocyclique ou hétérocyclique saturé, insaturé ou aromatique, monocyclique ou polycyclique; un enchaînement des groupes précités.

11. Procédé selon la revendication 10 **caractérisé par le fait que** le composé organique hydroxylé présente au moins

un groupe électro-attracteur (G) choisi parmi l'un des atomes ou groupes suivants :

. un groupe acyle ayant de 2 à 20 atomes de carbone, de préférence, acétyle,
. un groupe de formule :

$-X$
$-CX_3$
$-CF_2-CF_3$
$-[CF_2]_p-CF_3$
$-C_p H_a F_b$
$-COOM$
$-COOR_3$
$-CHO$
$-SO_3-M$
$-SO_3-R_3$
$-SO_2-R_3$
$-SO-CF_3$
$-SO_2-CF_3$
$-S-CF_3$
$-NO_2$
$-CN$
$-N=C(R_3)_2$
$-NH-CO-R_3$
$-N^+(R_3)_3$
$-P(O)(OR_3)_2$
$-Si(R_3)_3$

dans lesdites formules, les groupes $R_3$ identiques ou différents représentent un atome d'hydrogène ou un groupe alkyle linéaire ou ramifié, saturé ou insaturé ayant de 1 à 20 atomes de carbone ; X symbolise un atome d'halogène, de préférence un atome de chlore, de brome ou de fluor : M représente un atome de métal alcalin, de préférence le sodium ou le potassium : p représente un nombre allant de 1 à 10, b un nombre allant de 3 à 21 et a + b = 2 p + 1.

**12.** Procédé selon la revendication 11 **caractérisé par le fait que** le composé organique hydroxylé répond à la formule (I) dans laquelle $R_1$ représente :

- un groupe aliphatique acyclique linéaire ou ramifié,
- un groupe aliphatique acyclique porteur d'un substituant cyclique, éventuellement substitué, de préférence, un cycle benzénique, pouvant être relié au cycle par un lien valentiel, un hétéroatome ou un groupe fonctionnel,
- un groupe carbocyclique, saturé ou non ayant de préférence 5 ou 6 atomes de carbone dans le cycle ; un groupe hétérocyclique, saturé ou non, comportant notamment 5 ou 6 atomes dans le cycle dont 1 ou 2 hétéroatomes tels que les atomes d'azote, de soufre et d'oxygène ; un groupe carbocyclique ou hétérocyclique aromatique, monocyclique, de préférence, phényle ou pyridyle ou polycyclique condensé ou non, de préférence, naphtyle.

**13.** Procédé selon l'une des revendications 10 à 12 **caractérisé par le fait que** le composé organique hydroxylé répond à la formule (I) dans laquelle $R_1$ représente un groupe alkyle linéaire ou ramifié ayant de 1 à 12 atomes de carbone, de préférence de 1 à 6 atomes de carbone : la chaîne hydrocarbonée pouvant être éventuellement interrompue par un hétéroatome ou par un groupe fonctionnel et/ou porteuse d'un substituant.

**14.** Procédé selon la revendication 1 **caractérisé par le fait que** le composé organique hydroxylé répond à la formule (Ia) :

$$R_1 — O — H \qquad (Ia)$$

dans ladite formule (Ia), $R_1$ représente une chaîne alkyle fluorée ou perfluorée comprenant de 1 à 10 atomes de carbone et de 1 à 21 atomes de fluor, de préférence, de 3 à 21 atomes de fluor.

**15.** Procédé selon la revendication 1 **caractérisé par le fait que** le composé organique hydroxylé est choisi parmi : le 2,2,2-trifluoroéthanol, le 2,2-difluoroéthanol, le 1,1-difluoroéthanol, le pentafluoroéthanol, l'hexafluoroisopropanol, le pentafluorophénol, le p-nitrophénol, le p-trifluorométhylphénol.

**16.** Procédé selon la revendication 1 **caractérisé par le fait que** l'agent sulfonylant est un composé comprenant au moins un groupe sulfonyle de type -$SO_2$-$R_4$ dans lequel $R_4$ représente un groupe hydrocarboné ayant de 1 à 20 atomes de carbone.

**17.** Procédé selon la revendication 16 **caractérisé par le fait que** l'agent sulfonylant est un composé qui répond à la formule suivante (II) :

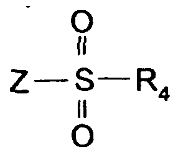

(II)

dans ladite formule (II) :

- $R_4$ représente un groupe hydrocarboné ayant de 1 à 20 atomes de carbone,
- Z représente :
  - . un groupe hydroxyle ou un atome d'halogène, de préférence, un atome de chlore ou de brome,
  - . un groupe -O-$SO_2$-$R_4$'·dans lequel $R_4$', identiques ou différents de $R_4$, a la signification donnée pour $R_4$.

**18.** Procédé selon la revendication 17 **caractérisé par le fait que** l'agent sulfonylant est un composé répondant à la formule (III) dans laquelle Z représente un atome de chlore ou de brome.

**19.** Procédé selon la revendication 17 **caractérisé par le fait que** l'agent sulfonylant est un composé répondant à la formule (III) dans laquelle $R_4$ représente :

- un groupe alkyle ayant de 1 à 10 atomes de carbone, de préférence, 1 à 4 atomes de carbone et plus préférentiellement un groupe méthyle ou éthyle, éventuellement porteur d'un atome d'halogène, d'un groupe $CF_3$ ou d'un groupe ammonium $N(R_5)_4$, $R_5$, identiques ou différents, représentant un groupe alkyle ayant de 1 à 4 atomes de carbone,
- un groupe cycloalkyle ayant de 3 à 8 atomes de carbone, de préférence, un groupe cyclohexyle,
- un groupe aryle ayant de 6 à 12 atomes de carbone, de préférence, un groupe phényle éventuellement porteur d'un groupe alkyle ayant de 1 à 10 atomes de carbone, de préférence, 1 à 4 atomes de carbone et plus préférentiellement un groupe méthyle ou éthyle, d'un atome d'halogène, d'un groupe $CF_3$ ou d'un groupe $NO_2$,
- un groupe $CX_3$ dans lequel X représente un atome de fluor, de chlore ou de brome,
- un groupe $CF_2$ - $CF_3$,
- un groupe $C_p$ $H_a$ $F_b$ dans lequel p représente un nombre allant de 1 à 10, b un nombre allant de 3 à 21 et a + b = 2 p + 1.

**20.** Procédé selon la revendication 17 **caractérisé par le fait que** l'agent sulfonylant est un composé répondant à la formule (III) dans laquelle le groupe -$SO_2$- $R_4$ représente :

- les tosyles (p-toluènesulfonyle) -$SO_2$-$C_6H_4$-$CH_3$
- les brosyles (p-bromobenzènesulfonyle) -$SO_2$-$C_6H_4$-Br
- les nosyles (p-nitrobenzènesulfonyle) -$SO_2$-$C_6H_4$-$NO_2$
- les mésyles (méthanesulfonyle) -$SO_2$-$CH_3$
- les bétyles (ammonioalcanesulfonyle) -$SO_2$-$(CH_2)_nNMe_3^+$ avec n compris entre 0 et 6,
- les triflyles (trifluorométhanesulfonyle) -$SO_2$-$CF_3$
- les nonaflyles (nonafluorobutanesulfonyle) -$SO_2$-$C_4F_9$
- les trésyles (2,2,2-trifluroéthanesulfonyle).-$SO_2$-$CH_2$-$CF_3$

**21.** Procédé selon la revendication 16 **caractérisé par le fait que** l'agent sulfonylant est choisi parmi :

- l'anhydride triflique,
- le chlorure de méthanesulfonyle,
- le chlorure de trifluorométhanesulfonyle,
- le chlorure de benzènesulfonyle,
- le chlorure de p-toluènesulfonyle.

**22.** Procédé selon l'une des revendications 1 à 21 **caractérisé par** le fait la réaction entre le composé hydroxylé et l'agent de sulfonylation, est conduite en phase liquide, en présence ou en l'absence d'un solvant organique de préférence, un solvant organique, aprotique et peu polaire.

**23.** Procédé selon l'une des revendications 1 à 22 **caractérisé par le fait que** le rapport entre le nombre de moles d'agent de sulfonylation et le nombre de moles de composé hydroxylé varie entre 0,9 et 2 et se situe de préférence entre 1,0 et 1,2.

**24.** Procédé selon l'une des revendications 1 à 23 **caractérisé par le fait que** le la quantité de catalyseur représente de 0,01 à 20 %, de préférence, de 0,05 à 10 %, et encore plus préférentiellement entre 0,1 et 2 % du poids du composé hydroxylé engagé.

**25.** Procédé selon l'une des revendications 1 à 24 **caractérisé par le fait que** la température à laquelle est mise en oeuvre la réaction de sulfonylation se situe entre 20°C et 150°C, de préférence entre 70°C et 100°C.

**26.** Procédé selon l'une des revendications 1 à 25 **caractérisé par le fait que** l'on charge l'agent de sulfonylation et le catalyseur de type acide de Lewis et l'on porte sous agitation, le mélange réactionnel à la température souhaitée puis l'on additionne ensuite, le composé hydroxylé, de préférence, progressivement.

**27.** Procédé selon l'une des revendications 1 à 26 **caractérisé par le fait que** l'on obtient un composé sulfonylé répondant à la formule (III) :

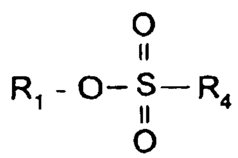

(III)

dans ladite formule (III), $R_1$ et $R_4$ ont la signification donnée précédemment

**28.** Procédé selon l'une des revendications 1 à 27 **caractérisé par le fait que** l'on obtient le 2,2,2-trifluoroéthylméthanesulfonate, à partir du 2,2,2-trifluoroéthanol et du chlorure de mésyle, en présence de chlorure d'antimoine M.

**29.** Procédé de préparation d'un composé organique hydroxylé répondant à la formule (VI) :

$$R_6 — O — R_1 \qquad (VI)$$

dans ladite formule $R_6$, identique ou différent de $R_1$, a les significations données pour $R_1$, sans toutefois nécessité la présence d'un groupe électro-attracteur, **caractérisé par le fait qu'** il consiste :

- à préparer un composé de formule (III) obtenu selon le procédé décrit dans l'une des revendications 1 à 28,
- à faire réagir en présence d'une base, le composé de formule (III) avec un composé organique hydroxylé de formule (V) :

$$R_6 — O — H \qquad (V)$$

dans ladite formule $R_6$ a la signification donnée précédemment.

**30.** Procédé selon la revendication 29 **caractérisé par le fait que** l'on effectue la préparation d'un composé de formule (VI) dans laquelle $R_6$ représente un cycle aromatique et qui répond à la formule suivante :

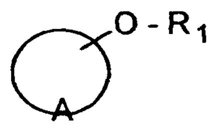

(VIa)

dans ladite formule (VIa), $R_1$ a la signification donnée précédemment et A symbolise le reste d'un cycle formant tout ou partie d'un système carbocyclique ou hétérocyclique, aromatique, monocyclique ou polycyclique.

**31.** Procédé selon la revendication 30 **caractérisé par le fait que** le composé aromatique hydroxylé répond à la formule (VIa) dans laquelle le reste A éventuellement substitué représente, le reste :

- d'un composé monocyclique, carbocyclique, aromatique, tel que par exemple, le benzène,
- d'un composé polycyclique, condensé, aromatique, tel que par exemple, le naphtalène,
- d'un composé polycyclique, non condensé, carbocyclique, aromatique, tel que par exemple, le phénoxybenzène,
- d'un composé polycyclique, condensé, carbocyclique, partiellement aromatique, tel que par exemple, le tétrahydronaphtalène, le 1,2-méthylène dioxybenzène,
- d'un composé polycyclique, non condensé, carbocyclique, partiellement aromatique, tel que par exemple, le cyclohexylbenzène,
- d'un composé monocyclique, hétérocyclique, aromatique, tel que par exemple, la pyridine, le furane, le thiophène,
- d'un composé polycyclique, condensé, aromatique, partiellement hétérocyclique, tel que, par exemple, la quinoléïne, l'indole ou le benzofurane,
- d'un composé polycyclique, non condensé, aromatique, partiellement hétérocyclique, tel que, par exemple, les phénylpyridines, les naphtylpyridines,
- d'un composé polycyclique, condensé, partiellement aromatique, partiellement hétérocyclique, tel que par exemple, la tétrahydroquinoléïne,
- d'un composé polycylique, non condensé, partiellement aromatique, partiellement hétérocyclique, tel que par exemple, la cyclohexylpyridine.

**32.** Procédé selon la revendication 29 **caractérisé par le fait que** la réaction du composé de formule (V) et du composé de formule (III) est une réaction effectuée en présence d'une base qui peut être une base minérale telle qu'un sel de métal alcalin, de préférence, un hydroxyde de métal alcalin qui peut être l'hydroxyde de sodium ou de potassium ; un carbonate de métal alcalin, de préférence, de potassium ; un hydroxyde d'ammonium quaternaire : un alcoolate métallique de préférence, le méthylate de sodium ou de potassium, le tert-butylate de potassium.

**33.** Procédé selon la revendication 29 **caractérisé par le fait que** la réaction a lieu en présence d'un solvant organique, de préférence, un solvant organique polaire et plus préférentiellement le DMAC et le DMF.

**34.** Procédé selon la revendication 29 **caractérisé par le fait que** la réaction entre le composé de formule (V) et le composé de formule (III) a lieu entre 60°C et 160°C, de préférence, entre 120°C et 140°C.

## Patentansprüche

**1.** Verfahren zur Sulfonylierung einer organischen Hydroxylverbindung, die mindestens eine elektronenziehende Gruppe aufweist, die der Hydroxylgruppe nahe genug ist, um sie zu deaktivieren; wobei die elektronenziehende Gruppe in der linearen oder cyclischen gesättigten oder ungesättigten Kohlenwasserstoffgruppe, die die Hydroxylgruppe trägt, oder auch an einem aromatischen Carbocyclus oder Heterocyclus, gebunden an ein Kohlenstoffatom, das mit demjenigen verknüpft oder demjenigen nahe ist, das die Hydroxylgruppe trägt, vorhanden sein kann, **dadurch gekennzeichnet, dass** es darin besteht, die Verbindung mit einem Sulfonylierungsmittel in Gegenwart einer wirksamen Menge einer Lewis-Säure reagieren zu lassen, die eine Verbindung ist, die ein metallisches oder

metallartiges Kation, Acceptor von Elektronenpaaren, umfasst, die in der von R. PEARSON definierten Klassifikation von "Härte (hart) und Weichheit (weich)" als "intermediär" betrachtet wird.

2. Verfahren nach Anspruch 1, **dadurch gekennzeichnet, dass** das erzeugte "intermediäre" Kation einen Oxidationsgrad von mindestens + 2, vorzugsweise von + 3, + 4 oder + 5 aufweist.

3. Verfahren nach einem der Ansprüche 1 und 2, **dadurch gekennzeichnet, dass** das Kation ein metallisches oder metallartiges Kation der metallischen oder metallartigen Elemente der Gruppen (IIb), (IVb), (Vb) und (VIb) des Periodensystems der Elemente ist.

4. Verfahren nach einem der Ansprüche 1 bis 3, **dadurch gekennzeichnet, dass** das Kation aus den folgenden Kationen ausgewählt ist: $Zn^{2+}$, $Sn^{2+}$, $Sn^{4+}$, $Sb^{5+}$, $Bi^{3+}$, $Te^{4+}$ und ferner vorzugsweise $Sb^{5+}$.

5. Verfahren nach einem der Ansprüche 1 bis 4, **dadurch gekennzeichnet, dass** die Lewis-Säure eine Verbindung ist, die ein Anion umfasst, das aus den folgenden Anionen ausgewählt ist: harte Anionen, wie $SO_4^{2-}$, $CH_3COO^-$, $C_6H_5COO^-$, $CH_3SO_3^-$, $CF_3SO_3^-$, $CF_3C_6H_4SO_3^-$, oder intermediäre Anionen, wie $Cl^-$, $Br^-$, $NO_2^-$, $SO_3^{2-}$.

6. Verfahren nach Anspruch 5, **dadurch gekennzeichnet, dass** das Anion aus $Cl^-$ oder $Br^-$ ausgewählt ist.

7. Verfahren nach einem der Ansprüche 1 bis 6, **dadurch gekennzeichnet, dass** die Lewis-Säure ein organisches Salz, wie Acetat, Propionat, Benzoat, Methansulfonat, Trifluormethansulfonat der metallischen oder metallartigen Elemente der zuvor genannten Gruppen des Periodensystems der Elemente und/oder ein anorganisches Salz, wie Chlor, Brom, Iod, Sulfat, Oxid, und entsprechende Produkte der metallischen oder metallartigen Elemente der vorgenannten Gruppen ist.

8. Verfahren nach Anspruch 1, **dadurch gekennzeichnet, dass** die Lewis-Säure aus den metallischen Halogeniden und bevorzugter aus dem Chlorid oder Bromid von Antimon(V), Zinn(II) oder Zinn(IV), Zink(II), Wismut(III) und Tellur(IV) ausgewählt ist.

9. Verfahren nach einem der Ansprüche 1 bis 8, **dadurch gekennzeichnet, dass** der Katalysator auf einem mineralischen oder organischen Träger abgelagert ist, der vorzugsweise aus den Metalloxiden, wie den Oxiden von Aluminium, Silicium, Titan und/oder Zirkonium, Tonen und insbesondere Kaolin, Talk oder Montmonrillonit oder auch aus den gegebenenfalls durch eine Salpetersäurebehandlung aktivierten Kohlen, Acetylenschwarz oder den organischen Polymeren, vorzugsweise den Polyvinyl- oder Polystyrolpolymeren ausgewählt ist.

10. Verfahren nach einem der Ansprüche 1 bis 9, **dadurch gekennzeichnet, dass** die organische Hydroxylverbindung der Formel (I) gehorcht:

$$R_1\text{-O-H} \quad \text{(I)}$$

wobei in der Formel (I):

- $R_1$ eine Kohlenwasserstoffgruppe darstellt, substituiert mit mindestens einer elektronenziehenden Gruppe (G), umfassend 1 bis 40 Kohlenstoffatome, die eine aliphatische acyclische gesättigte oder ungesättigte lineare oder verzweigte Gruppe; eine carbo- oder heterocyclische gesättigte, ungesättigte oder aromatische monocyclische oder polycyclische Gruppe; eine Verknüpfung der vorgenannten Gruppen sein kann.

11. Verfahren nach Anspruch 10, **dadurch gekennzeichnet, dass** die organische Hydroxylverbindung mindestens eine elektronenziehende Gruppe (G) aufweist, die aus einem der folgenden Atome oder Gruppen ausgewählt ist:

- eine Acylgruppe mit 2 bis 20 Kohlenstoffatomen, vorzugsweise Acetyl,
- eine Gruppe der Formel:

$-X$
$-CX_3$
$-CF_2\text{-}CF_3$

$-[CF_2]_p-CF_3$
$-C_pH_a F_b$
-COOM
$-COOR_3$
-CHO
$-SO_3-M$
$-SO_3-R_3$
$-SO_2-R_3$
$-SO-CF_3$
$-SO_2-CF_3$
$-S-CF_3$
$-NO_2$
-CN
$-N=C(R_3)_2$
$-NH-CO-R_3$
$-N^+(R_3)_3$
$-P(O)(OR_3)_2$
$-Si(R_3)_3$

wobei in den Formeln die Gruppen $R_3$, identisch oder verschieden; ein Wasserstoffatom oder eine lineare oder verzweigte gesättigte oder ungesättigte Alkylgrüppe mit 1 bis 20 Kohlenstoffatomen darstellen; X ein Halogenatom, vorzugsweise ein Chlor-, Brom- oder ein Fluoratom darstellt; M ein Alkalimetallatom, vorzugsweise Natrium oder Kalium darstellt; p eine Zahl von 1 bis 10 darstellt, b eine Zahl von 3 bis 21 darstellt und a+b=2p+1 bedeutet.

**12.** Verfahren nach Anspruch 11, **dadurch gekennzeichnet, dass** die organische Hydroxylverbindung der Formel (I) gehorcht, wobei $R_1$ folgendes darstellt:

- eine aliphatische acyclische lineare oder verzweigte Gruppe,
- eine aliphatische acyclische Gruppe, die einen cyclischen, gegebenenfalls substituierten Substituenten, vorzugsweise einen benzolischen Cyclus trägt, der über eine Valenzbindung mit einem Cyclus, einem Heteroatom oder einer funktioneller Gruppe verknüpft sein kann,
- eine carbocyclische gesättigte oder ungesättigte Gruppe mit vorzugsweise 5 oder 6 Kohlenwasserstoffatomen in dem Cyclus; eine heterocyclische gesättigte oder ungesättigte Gruppe, die insbesondere 5 oder 6 Atome in dem Cyclus umfasst, wovon 1 oder 2 Heteroatome, wie Stickstoff-, Schwefel- und Sauerstoffatome, sind; eine carbocyclische oder heterocyclische aromatische monocyclische, vorzugsweise phenolische oder pyridylische oder polycyclische, kondensierte oder nicht kondensierte Gruppe, vorzugsweise Naphthyl.

**13.** Verfahren nach einem der Ansprüche 10 bis 12, **dadurch gekennzeichnet, dass** die organische Hydroxylverbindung der Formel (I) gehorcht, wobei $R_1$ eine lineare oder verzweigte Alkylgruppe mit 1 bis 12 Kohlenstoffatomen, vorzugsweise 1 bis 6 Kohlenstoffatomen darstellt; die Kohlenwasserstoffkette gegebenenfalls mit einem Heteroatom oder mit einer funktionellen Gruppe unterbrochen sein kann und/oder einen Substituenten tragen kann.

**14.** Verfahren nach Anspruch 1, **dadurch gekennzeichnet, dass** die organische Hydroxylverbindung der Formel (Ia) gehorcht:

$$R_1\text{-O-H} \qquad \text{(Ia)}$$

wobei in der Formel (Ia) $R_1$ eine fluorierte oder perfluorierte Alkylkette darstellt, die 1 bis 10 Kohlenstoffatome und 1 bis 21 Fluoratome, vorzugsweise 3 bis 21 Fluoratome umfasst.

**15.** Verfahren nach Anspruch 1, **dadurch gekennzeichnet, dass** die organische Hydroxylverbindung ausgewählt ist aus: 2,2,2-Trifluorethanol, 2,2-Difluorethanol, 1,1-Difluorethanol, Pentafluorethanol, Hexafluorisopropanol, Pentafluorphenol, p-Nitrophenol, p-Trifluormethylphenol.

**16.** Verfahren nach Anspruch 1, **dadurch gekennzeichnet, dass** das Sulfonylierungsmittel eine Verbindung ist, die mindestens eine Sulfonylgruppe des Typs-$SO_2$-$R_4$ umfasst, wobei $R_4$ eine Kohlenwasserstoffgruppe mit 1 bis 20

Kohlenstoffatomen darstellt.

**17.** Verfahren nach Anspruch 16, **dadurch gekennzeichnet, dass** das Sulfonylierungsmittel eine Verbindung ist, die der folgenden Formel (II) gehorcht:

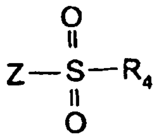

(II)

wobei in der Formel (II):

- $R_4$ eine Kohlenwasserstoffgruppe mit 1 bis 20 Kohlenstoffatomen darstellt;
- Z folgendes darstellt:
- eine Hydroxylgruppe oder ein Halogenatom, vorzugsweise ein Chlor- oder Bromatom,
- eine Gruppe-O-$SO_2$-$R_4$', wobei $R_4$', identisch oder verschieden von $R_4$, die für $R_4$ angegebene Bedeutung besitzt.

**18.** Verfahren nach Anspruch 17, **dadurch gekennzeichnet, dass** das Sulfonylierungsmittel eine Verbindung ist, die der Formel (III) gehorcht, wobei Z ein Chlor- oder Bromatom darstellt.

**19.** Verfahren nach Anspruch 17, **dadurch gekennzeichnet, dass** das Sulfonylierungsmittel eine Verbindung ist, die der Formel (III) gehorcht, wobei $R_4$ folgendes darstellt:

- eine Alkylgruppe mit 1 bis 10 Kohlenstoffatomen, vorzugsweise 1 bis 4 Kohlenstoffatomen, und bevorzugter eine Methyl- oder Ethylgruppe, die gegebenenfalls ein Halogenatom, ein Gruppe $CF_3$ oder eine Ammoniumgruppe $N(R_5)_4$ trägt, $R_5$, identisch oder verschieden, eine Alkylgruppe mit 1 bis 4 Kohlenstoffatomen darstellt,
- eine Cycloalkylgruppe mit 3 bis 8 Kohlenstoffatomen, vorzugsweise eine Cyclohexylgruppe,
- eine Arylgruppe mit 6 bis 12 Kohlenstoffatomen, vorzugsweise eine Phenylgruppe, die gegebenenfalls eine Alkylgruppe mit 1 bis 10 Kohlenstoffatomen, vorzugsweise 1 bis 4 Kohlenstoffatomen, und bevorzugter eine Methyl- oder Ethylgruppe, ein Halogenatom, eine Gruppe $CF_3$ oder einer Gruppe $NO_2$ trägt,
- eine Gruppe $CX_3$, wobei X ein Fluor-, Chlor- oder Bromatom darstellt,
- eine Gruppe $CF_2$-$CF_3$,
- eine Gruppe $C_pH_aF_b$, wobei p eine Zahl von 1 bis 10 darstellt, b eine Zahl von 3 bis 21 darstellt und a+b = 2p+1 bedeutet.

**20.** Verfahren nach Anspruch 17, **dadurch gekennzeichnet, dass** das Sulfonylierungsmittel eine Verbindung ist, die der Formel (III) gehorcht, wobei die Gruppe -$SO_2$-$R_4$ folgendes darstellt:

- Tosyle (p-Toluolsulfonyl) -$SO_2$-$C_6H_4$-$CH_3$
- Brosyle (p-Brombenzolsulfonyl) -$SO_2$-$C_6H_4$-Br
- Nosyle (p-Nitrobenzolsulfonyl) -$SO_2$-$C_6H_4$-$NO_2$
- Mesyle (Methansulfonyl) -$SO_2$-$CH_3$
- Betyle (Ammonioalkansulfonyl) -$SO_2$-$(CH_2)_n$$NMe_3^+$, wobei n zwischen 0 und 6 liegt,
- Triflyle (Trifluormethansulfonyl) -$SO_2$-$CF_3$
- Nonaflyle (Nonafluorbutansulfonyl) -$SO_2$-$C_4F_9$
- Tresyle (2,2,2-Trifluorethansulfonyl) -$SO_2$-$CH_2$-$CF_3$

**21.** Verfahren nach Anspruch 16, **dadurch gekennzeichnet, dass** das Sulfonylierungsmittel ausgewählt ist aus:

- Trifluormethansulfonsäureanhydrid,
- Methansulfonylchlorid,
- Trifluormethansulfonylchlorid,
- Benzolsulfonylchlorid,
- p-Toluolsulfonylchlorid.

**22.** Verfahren nach einem der Ansprüche 1 bis 21, **dadurch gekennzeichnet, dass** die Reaktion zwischen der Hydroxylverbindung und dem Sulfonylierungsmittel in flüssiger Phase in Gegenwart oder Abwesenheit eines organischen Lösungsmittels, vorzugsweise eines organischen aprotischen und wenig polaren Lösungsmittels durchgeführt wird.

**23.** Verfahren nach einem der Ansprüche 1 bis 22, **dadurch gekennzeichnet, dass** das Verhältnis zwischen der Anzahl von Molen an Sulfonylierungsmittel und der Anzahl von Molen an Hydroxylverbindung zwischen 0,9 und 2 schwankt und vorzugsweise zwischen 1,0 und 1,2 liegt.

**24.** Verfahren nach einem der Ansprüche 1 bis 23, **dadurch gekennzeichnet, dass** die Menge an Katalysator 0,01 bis 20 %, vorzugsweise 0,05 bis 10 % und noch bevorzugter zwischen 0,1 und 2 Gew.-% der betreffenden Hydroxylverbindung beträgt.

**25.** Verfahren nach einem der Ansprüche 1 bis 24, **dadurch gekennzeichnet, dass** die Temperatur, bei der die Sulfonylierungsreaktion durchgeführt wird, zwischen 20 °C und 150 °C, vorzugsweise zwischen 70 °C und 100 °C liegt.

**26.** Verfahren nach einem der Ansprüche 1 bis 25, **dadurch gekennzeichnet, dass** das Sulfonylierungsmittel und der Katalysator vom Typ einer Lewis-Säure vorgelegt werden und dass das Reaktionsgemisch unter Rühren auf die gewünschte Temperatur gebracht und anschließend die Hydroxylverbindung vorzugsweise schrittweise zugesetzt wird.

**27.** Verfahren nach einem der Ansprüche 1 bis 26, **dadurch gekennzeichnet, dass** eine sulfonylierte Verbindung erhalten wird, die der Formel (III) gehorcht:

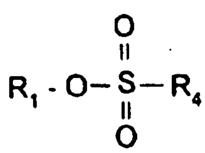

**(III)**

wobei in der Formel (III) $R_1$ und $R_4$ die zuvor gegebene Bedeutung besitzen.

**28.** Verfahren nach einem der Ansprüche 1 bis 27, **dadurch gekennzeichnet, dass** 2,2,2-Trifluorethylmethansulfonat ausgehend von 2,2,2-Trifluorethanol und Mesylchlorid in Gegenwart von Antimon(V)-chlorid erhalten wird.

**29.** Verfahren zur Herstellung einer organischen Hydroxylverbindung, die der Formel (VI) gehorcht:

$$R_6\text{-O-}R_1 \qquad (VI)$$

wobei in der Formel $R_6$, identisch oder verschieden von $R_1$, die für $R_1$ gegebenen Bedeutungen besitzt, ohne dass allerdings die Gegenwart einer elektronenziehenden Gruppe notwendig ist, **dadurch gekennzeichnet, dass** es besteht aus:

- Herstellen einer Verbindung der Formel (III), erhalten nach dem in einem der Ansprüche 1 bis 28 beschriebenen Verfahren,
- Reagieren lassen in Gegenwart einer Base der Verbindung der Formel (III) mit einer organischen Hydroxylverbindung der Formel (V):

$$R_6\text{-O-H} \qquad (V)$$

wobei in der Formel $R_6$ die zuvor gegebene Bedeutung besitzt.

30. Verfahren nach Anspruch 29, **dadurch gekennzeichnet, dass** die Herstellung einer Verbindung der Formel (VI) durchgeführt wird, wobei $R_6$ einen aromatischen Cyclus darstellt, der der folgenden Formel gehorcht:

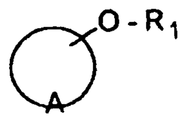

(VIa)

wobei in der Formel (VIa) $R_1$ die zuvor gegebene Bedeutung besitzt und A den Rest eines Cyclus darstellt, der ganz oder teilweise zu einem carbocyclischen oder heterocyclischen, aromatischen, monocyclischen oder polycyclischen System gehört.

31. Verfahren nach Anspruch 30, **dadurch gekennzeichnet, dass** die aromatische Hydroxylverbindung der Formel (VIa) gehorcht, wobei der Rest A, gegebenenfalls substituiert, den folgenden Rest darstellt:

- eine monocyclische, carbocyclische, aromatische Verbindung, wie beispielsweise Benzol,
- eine polycyclische, kondensierte aromatische Verbindung, wie beispielsweise Naphthalin,
- eine polycyclische, nicht kondensierte, carbocyclische, aromatische Verbindung, wie bespielsweise Phenoxybenzol,
- eine polycyclische, kondensierte, carbocyclische, teilweise aromatische Verbindung, wie beispielsweise Tetrahydronaphthalin, 1,2-Methylendioxybenzol,
- eine polycyclische, nicht kondensierte, carbocyclische, teilweise aromatische Verbindung, wie beispielsweise Cyclohexylbenzol,
- eine monocyclische, heterocyclische, aromatische Verbindung, wie beispielsweise Pyridin, Furan, Thiophen,
- eine polycyclische, kondensierte, aromatische, teilweise heterocyclische Verbindung, wie beispielsweise Chinolin, Indol oder Benzofuran,
- eine polycyclische, nicht kondensierte, aromatische, teilweise heterocyclische Verbindung, wie beispielsweise Phenylpyridin, Naphthylpyridin,
- eine polycyclische, kondensierte, teilweise aromatische, teilweise heterocyclische Verbindung, wie beispielsweise Tetrahydrochinolin,
- eine polycyclische, nicht kondensierte, teilweise aromatische, teilweise heterocyclische Verbindung, wie beispielsweise Cyclohexylpyridin.

32. Verfahren nach Anspruch 29, **dadurch gekennzeichnet, dass** die Umsetzung der Verbindung der Formel (V) und der Verbindung der Formel (III) eine Reaktion ist, die in Gegenwart einer Base durchgeführt wird, die eine mineralische Base, wie ein Alkalimetallsalz, vorzugsweise ein Alkalimetallhydroxid, das Natrium- oder Kaliumhydroxid sein kann; ein Alkalimetallcarbonat, vorzugsweise Kalium; ein quaternäres Ammoniumhydroxid; ein Metallalkoholat, vorzugsweise Natrium- oder Kaliummethylat, Kalium-tert-butylat sein kann.

33. Verfahren nach Anspruch 29, **dadurch gekennzeichnet, dass** die Umsetzung in Gegenwart eines organischen Lösungsmittels, vorzugsweise eines polaren, organischen Lösungsmittels und bevorzugter in DMAC und DMF erfolgt.

34. Verfahren nach Anspruch 29, **dadurch gekennzeichnet, dass** die Reaktion zwischen der Verbindung der Formel (V) und der Verbindung der Formel (III) zwischen 60 °C und 160 °C, vorzugsweise zwischen 120 °C und 140 °C stattfindet.

**Claims**

1. Method for sulphonylating a hydroxylated organic compound having at least one electron-withdrawing group sufficiently close to the hydroxyl group to deactivate it; it being possible for the electron-withdrawing group to be present on the linear or cyclic, saturated or unsaturated hydrocarbon-based chain carrying the hydroxyl group or else to be present on an aromatic carbocycle or heterocycle carried by a carbon atom that carries or is close to that which carries the hydroxyl group, **characterized in that** it consists in reacting said compound with a sulphonylating agent, in the presence of an effective amount of a Lewis acid, which is a compound comprising an electron-pair-acceptor metal or metalloid cation considered to be "borderline" in the "hard and soft" classification defined by R. Pearson.

2. Method according to Claim 1, **characterized in that** the "borderline" cation used has an oxidation state at least equal to +2, preferably equal to +3, +4 or +5.

3. Method according to either of Claims 1 and 2, **characterized in that** the cation is a metal or metalloid cation of the metal or metalloid elements of groups (IIb), (IVb), (Vb) and (VIb) of the Periodic Table of Elements.

4. Method according to one of Claims 1 to 3, **characterized in that** the cation is chosen from the following cations: $Zn^{2+}$, $Sn^{2+}$, $Sn^{4+}$, $Sb^{5+}$, $Bi^{3+}$ and $Te^{4+}$, and even more preferably $Sb^{5+}$.

5. Method according to one of Claims 1 to 4, **characterized in that** the Lewis acid is a compound comprising an anion chosen from the following anions: hard anions such as $SO_4^{2-}$, $CH_3COO^-$, $C_6H_5COO^-$, $CH_3SO_3^-$, $CF_3SO_3^-$ or $CF_3C_6H_4SO_3^-$, or borderline anions such as $Cl^-$, $Br^-$, $NO_2^-$ or $SO_3^{2-}$.

6. Method according to Claim 5, **characterized in that** the anion is chosen from $Cl^-$ or $Br^-$.

7. Method according to one of Claims 1 to 6, **characterized in that** the Lewis acid is an organic salt such as acetate, propionate, benzoate, methanesulphonate or trifluoromethanesulphonate of the metal or metalloid elements of the abovementioned groups of the Periodic Table of Elements, and/or an inorganic salt such as chloride, bromide, iodide, sulphate or oxide, and similar products, of the metal or metalloid elements of the abovementioned groups.

8. Method according to Claim 1, **characterized in that** the Lewis acid is chosen from metal halides, and more preferably antimony(V) chloride or bromide, tin(II) or (IV) chloride or bromide, zinc(II) chloride or bromide, bismuth(III) chloride or bromide and tellurium(IV) chloride or bromide.

9. Method according to one of Claims 1 to 8, **characterized in that** the catalyst is deposited on a mineral or organic support, preferably chosen from metal oxides, such as aluminium oxide, silicon oxide, titanium oxide and/or zirconium oxide, clays, and more particularly kaolin, talc or montmorillonite, or else from charcoals optionally activated by treatment with nitric acid, acetylene black or organic polymers, preferably polyvinyl or polystyrene polymers.

10. Method according to one of Claims 1 to 9, **characterized in that** the hydroxylated organic compound corresponds to formula (I):

$$R_1—O—H \qquad (I)$$

in said formula (I):

- $R_1$ represents a hydrocarbon-based group, substituted with at least one electron-withdrawing group (G), containing from 1 to 40 carbon atoms, which may be a saturated or unsaturated, linear or branched, acyclic aliphatic group; a saturated, unsaturated or aromatic, monocyclic or polycyclic, carbocyclic or heterocyclic group; a series of the abovementioned groups.

11. Method according to Claim 10, **characterized in that** the hydroxylated organic compound has at least one electron-withdrawing group (G) chosen from one of the atoms or groups below:

. an acyl group having from 2 to 20 carbon atoms, preferably acetyl,
. a group of formula:

$-X$
$-CX_3$
$-CF_2-CF_3$
$-[CF_2]_p-CF_3$
$-C_pH_aF_b$

-COOM
-$COOR_3$
-CHO
-$SO_3$-M
-$SO_3$-$R_3$
-$SO_2$-$R_3$
-SO-$CF_3$
-$SO_2$-$CF_3$
-S-$CF_3$
-$NO_2$
-CN
-N=$C(R_3)_2$
-NH-CO-$R_3$
-$N^+(R_3)_3$
-P(O) $(OR_3)_2$
-$Si(R_3)_3$

in said formulae, the groups $R_3$, which may be identical or different, represent a hydrogen atom or a linear or branched, saturated or unsaturated alkyl group having from 1 to 20 carbon atoms; X symbolizes a halogen atom, preferably a chlorine, bromine or fluorine atom; M represents an atom of an alkali metal, preferably sodium or potassium; p represents a number ranging from 1 to 10, b a number ranging from 3 to 21, and a + b = 2p + 1.

**12.** Method according to Claim 11, **characterized in that** the hydroxylated organic compound corresponds to formula (I) in which $R_1$ represents:

- a linear or branched, acyclic aliphatic group,
- an acyclic aliphatic group carrying an optionally substituted cyclic substituent, preferably a benzene ring, that may be attached to the ring via a valency bond, a hetero atom or a functional group,
- a saturated or unsaturated carbocyclic group preferably having 5 or 6 carbon atoms in the ring; a saturated or unsaturated heterocyclic group containing in particular 5 or 6 atoms in the ring, including 1 or 2 hetero atoms such as nitrogen, sulphur and oxygen atoms; an aromatic, carbocyclic or heterocyclic group that is monocyclic, preferably phenyl or pyridyl, or that is polycyclic and fused or nonfused, preferably naphthyl.

**13.** Method according to one of Claims 10 to 12, **characterized in that** the hydroxylated organic compound corresponds to formula (I) in which $R_1$ represents a linear or branched alkyl group having from 1 to 12 carbon atoms, preferably from 1 to 6 carbon atoms, it being possible for the hydrocarbon-based chain to be optionally interrupted with a hetero atom or with a functional group and/or a group carrying a substituent.

**14.** Method according to Claim 1, **characterized in that** the hydroxylated organic compound corresponds to formula (Ia) :

$$R1\text{-}O\text{-}H \quad \text{(Ia)}$$

in said formula (Ia), $R_1$ represents a fluorinated or perfluorinated alkyl chain comprising from 1 to 10 carbon atoms and from 1 to 21 fluorine atoms, preferably from 3 to 21 fluorine atoms.

**15.** Method according to Claim 1, **characterized in that** the hydroxylated organic compound is chosen from: 2,2,2-trifluoroethanol, 2,2-difluoroethanol, 1,1-difluoroethanol, pentafluoroethanol, hexafluoroisopropanol, pentafluorophenol, p-nitrophenol and p-trifluoromethylphenol.

**16.** Method according to Claim 1, **characterized in that** the sulphonylating agent is a compound comprising at least one sulphonyl group of -$SO_2$-$R_4$ type in which $R_4$ represents a hydrocarbon-based group having from 1 to 20 carbon atoms.

**17.** Method according to Claim 16, **characterized in that** the sulphonylating agent is a compound that corresponds to formula (II) below:

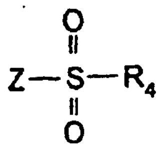

(II)

in said formula (II):

- $R_4$ represents a hydrocarbon-based group having from 1 to 20 carbon atoms,
- Z represents:

  - a hydroxyl group or a halogen atom, preferably a chlorine or bromine atom,
  - a group $-O-SO_2-R'_4$ in which $R'_4$, which may be identical to or different from $R_4$, has the meaning given for $R_4$.

**18.** Method according to Claim 17, **characterized in that** the sulphonylating agent is a compound corresponding to formula (II) in which Z represents a chlorine or bromine atom.

**19.** Method according to Claim 17, **characterized in that** the sulphonylating agent is a compound corresponding to formula (II) in which $R_4$ represents:

- an alkyl group having from 1 to 10 carbon atoms, preferably 1 to 4 carbon atoms, and more preferably a methyl or ethyl group, optionally carrying a halogen atom, a group $CF_3$ or an ammonium group $N(R_5)_4$, $R_5$, which may be identical or different, representing an alkyl group having from 1 to 4 carbon atoms,
- a cycloalkyl group having from 3 to 8 carbon atoms, preferably a cyclohexyl group,
- an aryl group having from 6 to 12 carbon atoms, preferably a phenyl group, optionally carrying an alkyl group having from 1 to 10 carbon atoms, preferably 1 to 4 carbon atoms, and more preferably a methyl or ethyl group, or carrying a halogen atom, a group $CF_3$ or a group $NO_2$,
- a group $CX_3$ in which X represents a fluorine, chlorine or bromine atom,
- a group $CF_2-CF_3$,
- a group $C_pH_aF_b$ in which p represents a number ranging from 1 to 10, b a number ranging from 3 to 21, and $a + b = 2p + 1$.

**20.** Method according to Claim 17, **characterized in that** the sulphonylating agent is a compound corresponding to formula (II) in which the group $-SO_2-R_4$ represents:

- the tosyls (p-toluenesulphonyl) $-SO_2-C_6H_4-CH_3$
- the brosyls (p-bromobenzenesulphonyl) $-SO_2-C_6H_4-Br$
- the nosyls (p-nitrobenzenesulphonyl) $-SO_2-C_6H_4-NO_2$
- the mesyls (methanesulphonyl) $-SO_2-CH_3$
- the betyls (ammonioalkanesulphonyl) $-SO_2-(CH_2)_nNMe_3^+$ with n between 0 and 6
- the triflyls (trifluoromethanesulphonyl) $-SO_2-CF_3$
- the nonaflyls (nonafluorobutanesulphonyl) $-SO_2-C_4F_9$
- the tresyls (2,2,2-trifluoroethanesulphonyl) $-SO_2-CH_2-CF_3$.

**21.** Method according to Claim 16, **characterized in that** the sulphonylating agent is chosen from:

- triflic anhydride,
- methanesulphonyl chloride,
- trifluoromethanesulphonyl chloride,
- benzenesulphonyl chloride,
- p-toluenesulphonyl chloride.

**22.** Method according to one of Claims 1 to 21, **characterized in that** the reaction between the hydroxylated compound and the sulphonylating agent is carried out in liquid phase, in the presence or in the absence of an organic solvent, preferably an aprotic and relatively nonpolar organic solvent.

**23.** Method according to one of Claims 1 to 22, **characterized in that** the ratio of the number of moles of sulphonylating agent to the number of moles of hydroxylated compound ranges between 0.9 and 2, and is preferably between 1.0 and 1.2.

**24.** Method according to one of Claims 1 to 23, **characterized in that** the amount of catalyst represents from 0.01 to 20%, preferably from 0.05 to 10%, and even more preferably between 0.1 and 2%, of the weight of the hydroxylated compound used.

**25.** Method according to one of Claims 1 to 24, **characterized in that** the temperature at which the sulphonylation reaction is carried out is between 20°C and 150°C, preferably between 70°C and 100°C.

**26.** Method according to one of Claims 1 to 25, **characterized in that** the sulphonylating agent and the catalyst of Lewis acid type are charged, and the reaction mixture is brought to the desired temperature, with stirring, then the hydroxylated compound is subsequently added, preferably gradually.

**27.** Method according to one of Claims 1 to 26, **characterized in that** a sulphonylated compound is obtained, corresponding to formula (III):

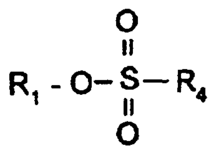

**(III)**

in said formula (III), $R_1$ and $R_4$ have the meaning given above.

**28.** Method according to one of Claims 1 to 27, **characterized in that** 2,2,2-trifluoroethylmethanesulphonate is obtained from 2,2,2-trifluoroethanol and mesyl chloride, in the presence of antimony(V) chloride.

**29.** Method for preparing a hydroxylated organic compound corresponding to formula (VI):

$$R_6\text{-O-}R_1 \qquad \text{(VI)}$$

in said formula $R_6$, which may be identical to or different from $R_1$, has the meanings given for $R_1$, without however any need for the presence of an electron-withdrawing group, **characterized in that** it consists:

- in preparing a compound of formula (III) obtained according to the method described in one of Claims 1 to 28,
- in reacting, in the presence of a base, the compound of formula (III) with a hydroxylated organic compound of formula (V):

$$R_6\text{-O-H} \qquad \text{(V)}$$

in said formula, $R_6$ has the meaning given above.

**30.** Method according to Claim 29, **characterized in that** the preparation of a compound of formula (VI) is carried out, in which $R_6$ represents an aromatic ring and which corresponds to the formula below:

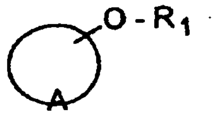

(VIa)

in said formula (VIa), $R_1$ has the meaning given above and A symbolizes the residue of a ring forming all or part of an aromatic, monocyclic or polycyclic, carbocyclic or heterocyclic system.

**31.** Method according to Claim 30, **characterized in that** the hydroxylated aromatic compound corresponds to formula (VIa) in which the optionally substituted residue A represents the residue:

- of an aromatic, carbocyclic, monocyclic compound, for instance benzene,
- of an aromatic, fused, polycyclic compound, for instance naphthalene,
- of an aromatic, carbocyclic, nonfused, polycyclic compound, for instance phenoxybenzene,
- of a partially aromatic, carbocyclic, fused, polycyclic compound, for instance tetrahydronaphthalene or 1,2-methylene dioxybenzene,
- of a partially aromatic, carbocyclic, nonfused, polycyclic compound, for instance cyclohexylbenzene,
- of an aromatic, heterocyclic, monocyclic compound, for instance, pyridine, furan or thiophene,
- of a partially heterocyclic, aromatic, fused, polycyclic compound, for instance quinoline, indole or benzofuran,
- of a partially heterocyclic, aromatic, nonfused, polycyclic compound, for instance phenylpyridines or naphthylpyridines,
- of a partially heterocyclic, partially aromatic, fused, polycyclic compound, for instance tetrahydroquinoline,
- of a partially heterocyclic, partially aromatic, nonfused, polycyclic compound, for instance cyclohexylpyridine.

**32.** Method according to Claim 29, **characterized in that** the reaction of the compound of formula (V) and of the compound of formula (III) is a reaction carried out in the presence of a base, which may be a mineral base such as an alkali metal salt, preferably an alkali metal hydroxide which may be sodium hydroxide or potassium hydroxide; an alkali metal carbonate, preferably potassium carbonate; a quaternary ammonium hydroxide; or a metal alkoxide, preferably sodium methoxide, potassium methoxide or potassium tert-butoxide.

**33.** Method according to Claim 29, **characterized in that** the reaction takes place in the presence of an organic solvent, preferably a polar organic solvent, and even more preferably DMAC and DMF.

**34.** Method according to Claim 29, **characterized in that** the reaction between the compound of formula (V) and the compound of formula (III) takes place at between 60°C and 160°C, preferably between 120°C and 140°C.